# EUROPÄISCHE PATENTSCHRIFT

(11) **EP 3 329 885 B1**
(45) Veröffentlichungstag und Bekanntmachung des Hinweises auf die Patenterteilung: **08.01.2020**
(21) Anmeldenummer: 16020480.6
(22) Anmeldetag: 05.12.2016
(51) Int. Cl.: A61F 9/008, A61F 9/009, A61B 3/00, A61B 3/10

(54) **OPHTHALMOLOGISCHE PATIENTEN-INTERFACEVORRICHTUNG**
OPHTHALMIC PATIENT INTERFACE DEVICE
DISPOSITIF D'INTERFACE OPHTALMOLOGIQUE POUR PATIENTS

(43) Veröffentlichungstag der Anmeldung: 06.06.2018
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- DE-A1-102010 022 298
- US-A1- 2004 243 111
- US-A1- 2004 243 113

## Beschreibung

### Technisches Gebiet

Die vorliegende Erfindung betrifft eine ophthalmologische Patienteninterfacevorrichtung und ein ophthalmologisches Lasersystem mit einer ophthalmologischen Patienteninterfacevorrichtung. Die vorliegende Erfindung betrifft insbesondere eine ophthalmologische Patienteninterfacevorrichtung mit einer Befestigungsvorrichtung, zum Befestigen der Patienteninterfacevorrichtung an einem Auge eines Patienten, und einer Kopplungsvorrichtung zum mechanischen Ankoppeln der Patienteninterfacevorrichtung an einen Applikationskopf eines ophthalmologischen Lasersystems.

### Stand der Technik

Die Behandlung von Augengewebe z.B. für Gewebeschnitte und Gewebeabbau erfolgt heutzutage typischerweise mittels Lasertechnologie. Insbesondere werden für die Gewebebearbeitung stark fokussierte Femtosekundenlaserpulse verwendet, die Pulsbreiten von typisch 100fs bis 1000fs (1fs= 10-15s) aufweisen. Zurzeit wird mit kommerziellen Femtosekundenlasersystemen die Cornea (Hornhaut) des Auges behandelt, insbesondere um refraktive Korrekturen der Hornhaut vorzunehmen. Da mit den Laserpulsen aber auch andere Gewebeteile und Gewebebereiche des Auges behandelt werden können, z.B. Sklera, Linse und Retina (Netzhaut), sind für die verschiedenen Anwendungsgebiete unterschiedliche Arbeitsabstände und damit unterschiedliche Fokusabstände erforderlich. Um unterschiedliche anwendungsspezifische Fokusdurchmesser, Fokusformen, Fokusausdehnungen in der Projektionsrichtung und/oder Strahldivergenzen zu erhalten, müssen ebenfalls verschiedene Projektionsoptiken mit jeweils unterschiedlicher Numerischer Apertur (NA) verwendet werden. Im Allgemeinen ist eine hohe NA wünschenswert, weil sich mit hoher NA kleine Brennpunkte (Spotgrösse) und damit eine kleinere Schnittzone pro Puls erzeugen lassen. Beim Einsatz am Auge mit kurzem Arbeitsabstand sind ein möglichst geringes Gewicht und eine kleine Baugrösse der Projektionsoptik anzustreben. Aufgrund der gewünschten kleinen Baugrösse, hohen NA, kurzen Arbeitsabstände und sehr kleinen Fokusgrössen (Spotgrössen) ist es allerdings kaum möglich Projektionsobjektive mit sogenannten Vario-Objektiven herzustellen, die einen grossen Arbeitsbereich, insbesondere das ganze Auge, abdecken können. Würde man den Arbeitsabstand der Projektionsobjektive vergrössern, liessen sich Vario-Objektive mit grossen Arbeitsbereichen einfacher konzeptionieren, doch der Aufwand an Kosten, Bauraum und Gewicht steigt überproportional mit Arbeitsabstand, Fokussierstärke und Tiefenbereich an, so dass solche Systeme ökonomisch nicht mehr vertretbar sind und die Gebrauchstauglichkeit deutlich eingeschränkt ist.

WO 2013/053367 beschreibt eine Laserbehandlungsvorrichtung und Schnittstellenvorrichtungen, die entfernbar an die Laserbehandlungsvorrichtung angekoppelt werden können. Jede der Schnittstellenvorrichtungen umfasst einen transparenten Kontaktkörper mit einer Auflagefläche zum Auflegen auf das Auge. Die Schnittstellenvorrichtungen weisen unterschiedliche optische Eigenschaften auf und verändern die Lage des Strahlfokus der Laserbehandlungsvorrichtung bezüglich der Auflagefläche. Mit anderen Worten, die Schnittstellenvorrichtungen gemäss WO 2013/053367 verschieben den Fokus der Laserbehandlungsvorrichtung, so dass dieser anwendungsspezifisch in einen unterschiedlichen Bereich des Auges zu liegen kommt. Die Schnittstellenvorrichtungen gemäss WO 2013/053367 sind nicht geeignet für Laserbehandlungssysteme mit Projektionsobjektiven, die während der Behandlung mechanische Scannbewegungen ausführen, da die Schnittstellenvorrichtungen zur Erhaltung gleichbleibender Abbildungseigenschaften (insbesondere der Spotgrösse) mit dem Projektionsobjektiv mitbewegt werden müssten und dabei das an der Auflagefläche kontaktierte Auge verletzen würden.

DE 10 2010 022298 beschreibt eine Vorrichtung für die Kataraktchirurgie, welche ein zurüstbares Modul aufweist, das schwenkbar an enem Mikroskop gehalten ist und definiert zwischen ein Mikroskopobjektiv und ein am Auge angedocktes Kontaktglas positionierbar ist. Gemäss DE 10 2010 022298 wird ein Laserstrahl in das Mikroskop und von dort aus in ein hochaperturiges Objektiv des zurüstbaren Moduls eingekoppelt. Durch Bewegung des hochaperturigen Objektivs wird der Fokus des Laserstrahls verstellt.

### Darstellung der Erfindung

Es ist eine Aufgabe der vorliegenden Erfindung eine ophthalmologische Patienteninterfacevorrichtung und ein ophthalmologisches Lasersystem mit einer ophthalmologischen Patienteninterfacevorrichtung vorzuschlagen, welche zumindest einige Nachteile des Stands der Technik nicht aufweisen. Es ist insbesondere eine Aufgabe der vorliegenden Erfindung eine ophthalmologische Patienteninterfacevorrichtung und ein ophthalmologisches Lasersystem mit einer ophthalmologischen Patienteninterfacevorrichtung vorzuschlagen, welche unterschiedliche Behandlungstiefen im Auge ermöglichen, ohne dafür den Fokus des ophthalmologischen Lasersystems verschieben zu müssen.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Patienteninterfacevorrichtung, welche eine Kopplungsvorrichtung zum mechanischen Ankoppeln der Patienteninterfacevorrichtung an einen Applikationskopf eines ophthalmologischen Lasersystems umfasst, überdies ein Linsensystem umfasst, das, im am Applikationskopf angekoppelten Zustand der Patienteninterfacevorrichtung, bei einer Behandlung eines Auges, zwischen dem Auge und dem Applikationskopf angeordnet ist, eingekoppelt in einen Strahlengang von einem Projektionsobjektiv des Applikationskopfes zum Auge. Dabei ist das Linsensystem so eingerichtet, dass es eine im Strahlengang dem Linsensystem vorgelagerte erste Fokalfläche des Projektionsobjektivs an eine im Strahlengang dem Linsensystem nachgeschaltete zweite Fokalfläche im Auge abbildet, derart, dass ein vom Projektionsobjektiv auf die erste Fokalfläche fokussierter Laserstrahl in der zweiten Fokalfläche im Auge eine Gewebebearbeitung bewirkt. Durch die Abbildung der Fokalfläche des Projektionsobjektivs durch das Linsensystem auf eine nachgelagerte Fokalfläche im Augengewebe, können mit dem ophthalmologischen Lasersystem unterschiedliche Behandlungstiefen im Auge erreicht werden, ohne dass dafür der Fokussierbereich des ophthalmologischen Lasersystems vergrössert werden muss. Die ophthalmologische Patienteninterfacevorrichtung ermöglicht eine kostengünstige Alternative zu grossen Vario-Objektiven und hat insbesondere den Vorteil, dass der Anwendungsbereich bestehender Systeme flexibel und einfach erweitert werden kann, ohne dass das bestehende System durch Neuentwicklungen ersetzt werden muss.

In einer Ausführungsvariante umfasst das Linsensystem eine Relais-Optik, welche eingerichtet ist, die erste Fokalfläche des Projektionsobjektivs auf die zweite Fokalfläche im Auge abzubilden.

In einer Ausführungsvariante ist das Linsensystem eingerichtet, einen ersten Bildbereich auf der ersten Fokalfläche des Projektionsobjektivs auf einen bezüglich des ersten Bildbereichs kleineren, zweiten Bildbereich auf der zweiten Fokalfläche im Auge abzubilden.

In einer Ausführungsvariante ist das Linsensystem eingerichtet, die erste Fokalfläche des Projektionsobjektivs auf die zweite Fokalfläche im Auge abzubilden, welche in einem Bereich von und mit Augenlinse bis und mit Retina liegt.

In einer Ausführungsvariante ist die Patienteninterfacevorrichtung so ausgestaltet, dass im am Applikationskopf angekoppelten Zustand der Patienteninterfacevorrichtung ein Zwischenraum zwischen dem Projektionsobjektiv des Applikationskopfes und dem Linsensystem besteht, welcher ein Gas, Gasgemisch oder Vakuum enthält, und dass die erste Fokalfläche des Projektionsobjektivs in den Zwischenraum zu liegen kommt.

In einer Ausführungsvariante ist das Linsensystem eingerichtet, die erste Fokalfläche des Projektionsobjektivs derart auf die zweite Fokalfläche im Auge abzubilden, dass Aberrationen korrigiert werden, die aufgrund einer Fokussierung des Laserstrahls auf die erste Fokalfläche im Zwischenraum mit Gas, Gasgemisch oder Vakuum anstatt einer Fokussierung des Laserstrahls auf die erste Fokalfläche in Augengewebe auftreten.

In einer Ausführungsvariante umfasst die ophthalmologische Patienteninterfacevorrichtung optisch erkennbare Referenzmarkierungen, welche im Zwischenraum angeordnet sind.

In einer Ausführungsvariante ist die Patienteninterfacevorrichtung so ausgestaltet, dass, im am Applikationskopf angekoppelten Zustand der Patienteninterfacevorrichtung, das Projektionsobjektiv des Applikationskopfes über dem Linsensystem mechanisch frei verfahrbar ist.

In einer Ausführungsvariante umfasst die ophthalmologische Patienteninterfacevorrichtung eine Befestigungsvorrichtung mit einer oder mehreren Unterdruckkammern zum Befestigen der Patienteninterfacevorrichtung am Auge, und das Linsensystem ist auswechselbar mit der Befestigungsvorrichtung verbunden.

In einer Ausführungsvariante umfasst die ophthalmologische Patienteninterfacevorrichtung eine transparente Schutzbarriere, die, im am Auge befestigten Zustand der Patienteninterfacevorrichtung, zwischen dem Auge und dem Linsensystem angeordnet ist.

Neben der ophthalmologischen Patienteninterfacevorrichtung betrifft die vorliegende Erfindung überdies ein ophthalmologisches Lasersystem, das eine ophthalmologische Patienteninterfacevorrichtung gemäss einer der obenstehenden Ausführungsvarianten, eine Laserquelle zur Erzeugung eines Laserstrahls, ein Projektionsobjektiv zur fokussierten Projektion des Laserstrahls auf einen Fokus auf der ersten Fokalfläche, und eine Scannvorrichtung zum Bewegen des Fokus umfasst. Das ophthalmologische Lasersystem umfasst zudem einen Konfokaldetektor, welcher in den Strahlengang zwischen Laserquelle und Scannvorrichtung eingekoppelt ist, und welcher eingerichtet ist, eine Reflexion des Laserstrahls, durch in der zweiten Fokalfläche liegendes Augengewebe, zu erfassen.

In einer Ausführungsvariante umfasst das ophthalmologische Lasersystem einen Prozessor, der eingerichtet ist, das ophthalmologische Lasersystem derart zu steuern, dass die Laserquelle mit reduzierter Leistung betrieben wird, die keine Gewebeverarbeitung bewirkt, und dass die Scannvorrichtung den Fokus bei der reduzierten Leistung gemäss einem definierten Scannmuster bewegt, und vom Konfokaldetektor erfasste Werte der Reflexionen zugeordnet zu Punkten des Scannmusters zu speichern.

In einer Ausführungsvariante umfasst das ophthalmologische Lasersystem ein optisches Messsystem, welches eingerichtet ist, einen auf der zweiten Fokalfläche angeordneten Arbeitsbereich optisch entlang des Strahlengangs vom Projektionsobjektiv zum Auge zu ermitteln, wobei das optische Messsystem eine Kamera, die eingerichtet ist, ein Aufsichtsbild des Arbeitsbereichs zu erfassen, und/oder eine OCT-Vorrichtung umfasst, welche eingerichtet ist, den Arbeitsbereich durch optische Kohärenztomografie zu ermitteln.

In einer Ausführungsvariante umfasst das ophthalmologische Lasersystem eine im Strahlengang dem Linsensystem der ophthalmologischen Patienteninterfacevorrichtung vorgelagerte Adaptionsoptik, welche eingerichtet ist, Aberrationen zu korrigieren, die bei einer Abbildung eines Fokus des Laserstrahls von der ersten Fokalfläche auf die zweite Fokalfläche auftreten, wobei die Adaptionsoptik einen oder mehrere deformierbare Spiegel, in den Strahlengang einbringbare Platten, LCD-Spiegel, und/oder Linsensysteme umfasst.

In einer Ausführungsvariante umfasst das ophthalmologische Lasersystem ein optisches Messsystem, welches eingerichtet ist, eine Pupille des Auges optisch zu erfassen, einen Prozessor, der eingerichtet ist, eine Pupillengrösse zu ermitteln, und eine im Strahlengang dem Linsensystem der ophthalmologischen Patienteninterfacevorrichtung vorgelagerte Beam-Expander-Vorrichtung, welche eingerichtet ist, eine Strahlbreite des Laserstrahls abhängig von der Pupillengrösse anzupassen.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:

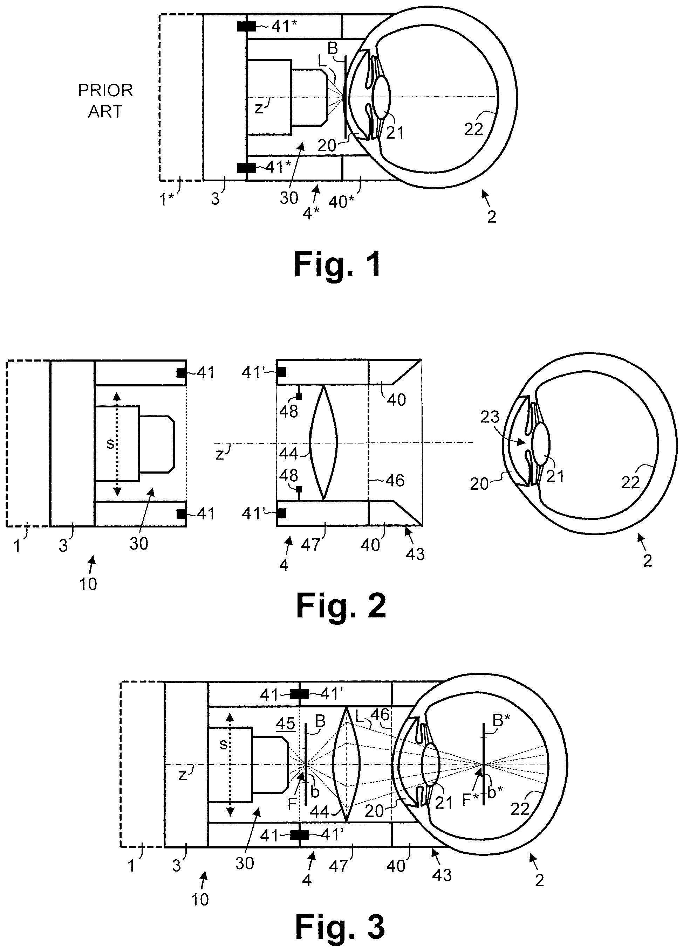

- Figur 1:: zeigt schematisch im Querschnitt eine ophthalmologische Patienteninterfacevorrichtung gemäss dem Stand der Technik, die an einem Auge eines Patienten befestigt und über eine Kopplungsvorrichtung mechanisch an einem Applikationskopf angekoppelt ist.
- Figur 2:: zeigt schematisch im Querschnitt einen Applikationskopf und eine an den Applikationskopf mechanisch ankoppelbare ophthalmologische Patienteninterfacevorrichtung mit einem Linsensystem, welche an einem Auge eines Patienten befestigbar ist.
- Figur 3:: zeigt schematisch im Querschnitt ein ophthalmologisches Lasersystem mit einem Applikationskopf und einer an den Applikationskopf mechanisch angekoppelten ophthalmologischen Patienteninterfacevorrichtung mit einem Linsensystem, welche an einem Auge eines Patienten befestigt ist.

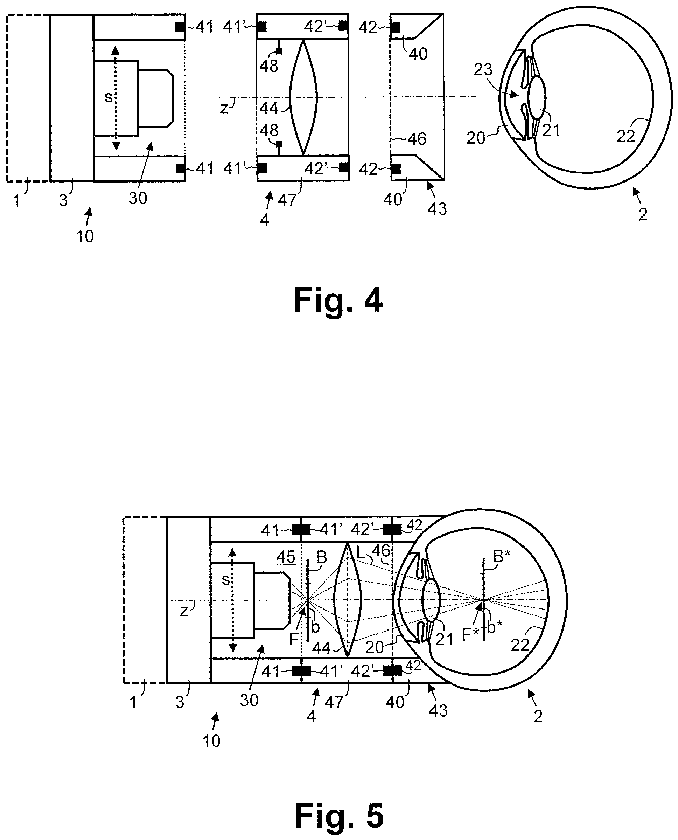

- Figur 4:: zeigt schematisch im Querschnitt einen Applikationskopf, eine an den Applikationskopf mechanisch ankoppelbare ophthalmologische Patienteninterfacevorrichtung mit einem Linsensystem, und eine an die ophthalmologische Patienteninterfacevorrichtung mechanisch ankoppelbare Befestigungsvorrichtung, mit der die Patienteninterfacevorrichtung am Auge eines Patienten befestigbar ist.
- Figur 5:: zeigt schematisch im Querschnitt ein ophthalmologisches Lasersystem mit einem Applikationskopf, einer an den Applikationskopf mechanisch angekoppelten ophthalmologischen Patienteninterfacevorrichtung mit einem Linsensystem, welche mechanisch angekoppelt an eine Befestigungsvorrichtung am Auge eines Patienten befestigt ist.

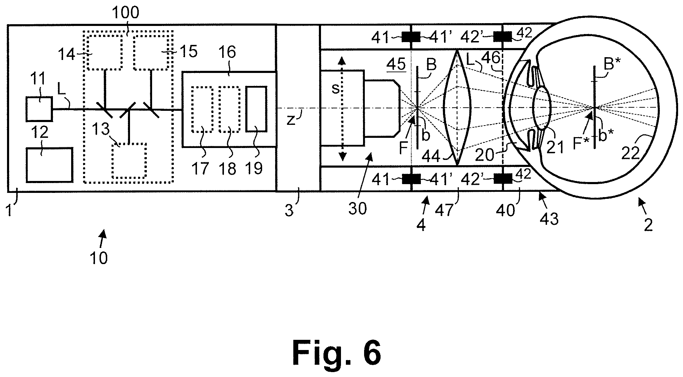

- Figur 6:: zeigt schematisch im Querschnitt ein ophthalmologisches Lasersystem mit einer Laservorrichtung, einem Applikationskopf, einer an den Applikationskopf mechanisch angekoppelten ophthalmologischen Patienteninterfacevorrichtung mit einem Linsensystem, welche mechanisch angekoppelt an eine Befestigungsvorrichtung am Auge eines Patienten befestigt ist, wobei mehrere Module der Laservorrichtung mittels eines Blockdiagramms dargestellt sind.

### Wege zur Ausführung der Erfindung

In den Figuren 1 bis 6 bezieht sich das Bezugszeichen 2 auf ein Auge eines Patienten, wobei die Cornea (Hornhaut) 20, die Linse 21, die Retina (Netzhaut) 22, und in den Figuren 2 und 4 die Pupille 23 jeweils mit einem eigenen Bezugszeichen versehen sind. In den Figuren 3, 5 und 6 ist die Pupille im Vergleich zu den Figuren 1, 2 und 4 in erhöht aufgeweitetem Zustand dargestellt, was beispielsweise durch Applikation pupillenaufweitender Substanzen erreicht wird.

Figur 1 illustriert schematisch im Querschnitt eine ophthalmologische Patienteninterfacevorrichtung 4* gemäss dem Stand der Technik, die an einem Ende mit einer Kopplungsvorrichtung 41* mechanisch an einem Applikationskopf 3 einer ophthalmologischen Laservorrichtung 1* angekoppelt ist und am anderen Ende mittels einer oder mehreren Unterdruckkammern 40* am Auge 2 befestigt ist. Bei der Behandlung des Auges 2 ist der Applikationskopf 3 mittels der Patienteninterfacevorrichtung 4* am Auge 2 fixiert und ein in der ophthalmologischen Laservorrichtung 1* erzeugter Laserstrahl L wird von einem Projektionsobjektiv 30 des Applikationskopfes 3 auf eine Fokalfläche B ins Auge 2 gestrahlt, im Beispiel der Figur 1 auf eine in der Cornea 20 verlaufende Fokalfläche B des Projektionsobjektivs 30.

In den Figuren 2 bis 6 bezieht sich das Bezugszeichen 10 auf ein ophthalmologisches Lasersystem, das eine Laservorrichtung 1 und einen Applikationskopf 3 mit einem Projektionsobjektiv 30 umfasst. Der Applikationskopf 3 ist beispielsweise mit einem festen oder beweglichen Tragarm an der Laservorrichtung 1 angebracht. In einer Ausführungsvariante ist das Projektionsobjektiv 30 des Applikationskopfes 3 mit einem Antrieb gekoppelt und wird in einer zur Projektionsrichtung z normal verlaufenden Scannebene s mechanisch verfahren (in x/y-Richtungen), wie mit dem Doppelpfeil s angedeutet ist. Wie in den Figuren 2-6 ersichtlich ist, weist der Applikationskopf 3 eine Kopplungsvorrichtung 41 auf, die eingerichtet ist, eine Patienteninterfacevorrichtung 4 mechanisch an den Applikationskopf 3 anzukoppeln und dadurch am Applikationskopf 3 zu befestigen. Die Patienteninterfacevorrichtung 4 weist dazu eine entsprechende Kopplungsvorrichtung 41' auf. Die Kopplungsvorrichtungen 41, 41' sind beispielsweise miteinander verschraubbar, einrastbar, einklinkbar, magnetisch oder durch Unterdruck (Vakuum) gekoppelt, oder werden bajonettverschlussartig mechanisch miteinander trennbar verbunden.

Im Zustand, in welchem die Patienteninterfacevorrichtung 4 am Applikationskopf 3 angekoppelt ist, wird ein Zwischenraum 45 zwischen dem Projektionsobjektiv 30 und dem Linsensystem 44 gebildet. Abhängig von der Ausführungsvariante enthält dieser Zwischenraum 45 ein Gas, ein Gasgemisch, z.B. Luft, oder ein Vakuum (Dichtungen, Pumpen und Leitungen, zur Erzeugung eines Vakuums oder der Einfüllung eines anderen Gases oder Gasgemisches als Luft, sind in den Figuren nicht dargestellt). Durch die Ankopplung der Patienteninterfacevorrichtung 4 am Applikationskopf 3 wird die Patienteninterfacevorrichtung 4 bezüglich des ophthalmologischen Lasersystems 10 in eine feste Position gesetzt. Wie in den Figuren 2 und 4 illustriert ist, weist die Patienteninterfacevorrichtung 4 optisch erkennbare Referenzmarkierungen 48 auf, die im Zwischenraum 45 angeordnet sind, beispielsweise Markierungen auf transparenten Trägern, die am Linsenträger 47 befestigt sind und von einem optischen Messsystem 100 erfasst und von einem Prozessor 12 als geometrisch bekannte Referenzpunkte verwendet werden können.

In einer Ausführungsvariante ist die Patienteninterfacevorrichtung 4 respektive der Zwischenraum 45 so ausgestaltet, dass, im Zustand, in welchem die Patienteninterfacevorrichtung 4 am Applikationskopf 3 angekoppelt ist, das Projektionsobjektiv 30 des Applikationskopfes 3 im Zwischenraum 45 über dem Linsensystem 44 mechanisch frei in der Scannebene s verfahrbar ist, wie mit dem Doppelpfeil s angedeutet ist.

Wie in der Figur 6 schematisch dargestellt ist, umfasst die Laservorrichtung 1 mehrere Komponenten und funktionale Module, insbesondere eine Laserquelle 11 und ein optisches Übertragungssystem 16 zur Übertragung eines in der Laserquelle 11 erzeugten Laserstrahls L von der Laserquelle 11 zum Projektionsobjektiv 30. Die Laserquelle 11 ist eingerichtet einen gepulsten Laserstrahl L zu erzeugen, insbesondere Femtosekundenlaserpulse. Das optische Übertragungssystem 16 umfasst eine Scannvorrichtung 19, die eingerichtet ist den Laserstrahl L respektive dessen Laserpulse zur Erzeugung von einem oder mehreren Scannmustern abzulenken. Die Scannvorrichtung 19 umfasst einen oder mehrere auslenkbare Ablenkspiegel, die mit einem Antrieb gekoppelt sind, z.B. ein Piezo- oder Galvanoantrieb. Der abgelenkte Laserstrahl L wird vom Projektionsobjektiv 30 auf eine Fokusfläche B fokussiert, wo der Fokus F des Laserstrahls L entsprechend der Ablenkung durch die Scannvorrichtung 19 gemäss dem Scannmuster in einem Bildbereich b bewegt wird.

Abhängig von der Ausführungsvariante umfasst das optische Übertragungssystem 16 optional eine Beam-Expander-Vorrichtung 17 und/oder eine Adaptionsoptik 18, deren Funktionen später beschrieben werden.

Abhängig von der Ausführungsvariante umfasst die Laservorrichtung 1 zudem optional ein optisches Messsystem 100 mit einem Konfokaldetektor 13, einer Kamera 14 und/oder einer OCT-Vorrichtung 15, deren Funktionen ebenfalls später beschrieben werden.

Zur Steuerung der Laservorrichtung 1 und deren Komponenten und funktionalen Module sind zudem ein oder mehrere Prozessoren 12 oder andere programmierte/konfigurierte elektronische Schaltkreis vorgesehen.

In den Figuren 2 bis 6 bezieht sich das Bezugszeichen 4 auf eine ophthalmologische Patienteninterfacevorrichtung, die einen Linsenträger 47 mit einem daran angebrachten Linsensystem 44 umfasst. Wie obenstehend beschrieben, weist die Patienteninterfacevorrichtung eine Kopplungsvorrichtung 41' zur mechanischen Ankopplung der Patienteninterfacevorrichtung 4 an den Applikationskopf 3 auf.

Die ophthalmologische Patienteninterfacevorrichtung 4 umfasst zudem eine Befestigungsvorrichtung 43 zum Befestigen der Patienteninterfacevorrichtung 4 an einem Auge 2 eines Patienten. Die Befestigungsvorrichtung 43 ist beispielsweise als Saugring ausgestaltet und umfasst eine oder mehrere Unterdruckkammern 40 zum Fixieren der Patienteninterfacevorrichtung 4 am Auge 2 durch Unterdruck (Vakuumpumpen und Saugleitungen sind in den Figuren nicht dargestellt).

In den Ausführungsvarianten der Figuren 2 und 3 ist die Befestigungsvorrichtung 43 fest mit dem Linsenträger 47 und dem daran angebrachten Linsensystem 44 verbunden. Die Figur 2 zeigt das ophthalmologische Lasersystem 10 und die Patienteninterfacevorrichtung 4 voneinander getrennt und losgelöst vom Auge 2. In der Figur 3 ist die Patienteninterfacevorrichtung 4 mechanisch am Applikationskopf 3 des ophthalmologischen Lasersystems 10 angekoppelt und mittels der Befestigungsvorrichtung 43 am Auge fixiert dargestellt. In den Ausführungsvarianten der Figuren 4 bis 6 sind die Befestigungsvorrichtung 43 und der Linsenträger 47 mit dem daran angebrachten Linsensystem 44 über Kopplungsvorrichtungen 42, 42' trennbar miteinander verbunden. Wie in den Figuren 4 bis 6 schematisch dargestellt ist, umfassen die Befestigungsvorrichtung 43 und der Linsenträger 47 dazu entsprechende Kopplungsvorrichtungen 42, 42', die wie obenstehend, im Zusammenhang mit den Kopplungsvorrichtungen 41, 41' zur Befestigung der Patienteninterfacevorrichtung 4 am Applikationskopf 3, beschrieben ausgestaltet sind. In einer Ausführungsvariante sind die Kopplungsvorrichtungen 42 der Befestigungsvorrichtung 43 entsprechend den Kopplungsvorrichtungen 41 des Applikationskopfes 3 so ausgestaltet, dass bei entferntem Linsenträger 47 und Linsensystem 44 der Applikationskopf 3 direkt an die Befestigungsvorrichtung 43 angekoppelt und dadurch direkt mit dem Befestigungsvorrichtung 43 verbunden und am Auge 2 befestigt werden kann. Die Befestigungsvorrichtung 43 ist insbesondere in dieser Ausführung als wegwerfbares Produkt vorgesehen, dass nach der Verwendung an einem Patienten, auch aus hygienischen und medizinisch präventiven Gründen, entsorgt wird. Die Figur 4 zeigt das ophthalmologische Lasersystem 10 und den Linsenträger 47 und die Befestigungsvorrichtung 43 der Patienteninterfacevorrichtung 4 voneinander getrennt und losgelöst vom Auge 2. In den Figuren 5 und 6 sind der Linsenträger 47 und die Befestigungsvorrichtung 43 der Patienteninterfacevorrichtung 4 miteinander verbunden, mechanisch am Applikationskopf 3 des ophthalmologischen Lasersystems 10 angekoppelt und mittels der Befestigungsvorrichtung 43 am Auge fixiert dargestellt.

Die Figuren 3, 5 und 6 illustrieren den Strahlengang des Laserstrahls L vom Projektionsobjektiv 30 ins Auge 2 bei eingekoppeltem Linsensystem 44 der Patienteninterfacevorrichtung 4. Wie aus den Figuren 3, 4 und 5 ersichtlich ist, ist das Linsensystem 44 im Strahlengang dem Projektionsobjektiv 30 nachgeschaltet und zwar nach der Fokalfläche B des Projektionsobjektivs 30, welche im Zwischenraum 45 liegt. Das Linsensystem 44 umfasst eine oder mehrere optische Linsen. Das Linsensystem 44 umfasst insbesondere eine Relais-Optik, die eingerichtet ist, die Fokalfläche B des Projektionsobjektivs 30 auf die Fokalfläche B* im Auge 2 abzubilden. Mit anderen Worten, der vom Projektionsobjektiv 30 auf die Fokalfläche B fokussierte Laserstrahl L (Fokus F), der sich nach der Fokalfläche B ausgehend vom Fokus F wieder ausweitet, wird vom Linsensystem 44 auf die Fokalfläche B* fokussiert (Fokus F*). Bei einer Einstellung der Laserquelle 11 auf eine Leistung zur Gewebebearbeitung wird durch Abbildung des Fokus F (auf der Fokalfläche B des Projektionsobjektivs 30) auf den Fokus F* (auf der Fokalfläche B* im Auge 2) eine Gewebebearbeitung im Auge 2 bewirkt. Die Relais-Optik ist bezüglich der Abbildung einer ersten Ebene auf eine andere zweite Ebene optimiert. Die Relais-Optik ist dabei insbesondere eingerichtet, die erste Ebene, hier Fokalfläche B, ohne Aberrationen respektive mit möglichst geringfügigen Aberrationen auf die andere zweite Ebene, hier Fokalfläche B*, abzubilden.

In einer Ausführungsvariante ist das Linsensystem 44 respektive die Relais-Optik eingerichtet, einen Bildbereich b auf der Fokalfläche B des Projektionsobjektivs 30 auf einen vergleichsweise kleineren Bildbereich b* auf der Fokalfläche B* im Auge 2 abzubilden. Dadurch wird der durch den Fokus F auf der Fokalfläche B gebildete Spot (Spotdurchmesser d) auf einen vergleichsweise kleineren Spot (Spotdurchmesser d*) des Fokus F* auf der Fokalfläche B* im Auge 2 abgebildet, wodurch die lokale Energiedichte im Fokus F* auf der Fokalfläche B* im Auge 2 erhöht wird. Diese Ausführungsvariante ist insbesondere für den Fall vorteilhaft, wo das Lasersystem 10 für die Chirurgie an der Linse 21 ausgelegt ist und entsprechend niedrige Fokussierkraft (NA) besitzt. Mit einem entsprechend ausgelegten Linsensystem 44 der Patienteninterfacevorrichtung 4 wird dabei nämlich ermöglicht, ein solches Lasersystem 10 mit erhöhter Fokussierkraft für die Bearbeitung der Cornea 20 einzusetzen.

In einer weiteren Ausführungsvariante ist das Linsensystem 44 respektive die Relais-Optik eingerichtet, Aberrationen zu korrigieren, die bei der "Zwischen"-Fokussierung des Laserstrahls L auf die Fokalfläche B des Projektionsobjektivs 30 deshalb entstehen, weil die Fokalfläche B anstatt wie in der Anordnung gemäss Figur 1 nicht im Augengewebe liegt, z.B. in der Cornea 20, oder im Flüssigkeitsvolumen eines flüssigkeitsbasierten Patienteninterfaces, sondern stattdessen ausserhalb des Auges 2 im Zwischenraum 45 liegt, der ein Gas, ein Gasgemisch oder ein Vakuum enthält.

Im Vergleich zu der Anordnung gemäss dem in Figur 1 dargestellten Stand der Technik, ermöglicht die in den Strahlengang zwischen Projektionsobjektiv 30 und Auge 2 eingekoppelte Patienteninterfacevorrichtung 4 mit dem Linsensystem 44 eine Abbildung respektive Verschiebung der Fokalfläche B, die beispielsweise mit der Anordnung gemäss dem Stand der Technik in die Cornea 20 bzw. in die Vorderkammer des Auges 2 zu liegen kommt (die Vorderkammer erstreckt sich von der Cornea 20 bis zur hinteren Linsenfläche der Linse 21), auf die Fokalfläche B* in tieferliegende Strukturen des Auges 2, im Bereich von und mit der Linse 21 bis und mit der Retina 22, ohne dazu Fokusverstellungen in der Laservorrichtung 1 oder im Projektionsobjektiv 30 vornehmen zu müssen, und ohne dass dabei der Fokussierbereich der Laservorrichtung 1 respektive des Projektionsobjektivs 30 erweitert werden muss. So kann beispielsweise ein Lasersystem 10, das für die Vorderkammerchirurgie eingerichtet ist, durch Zwischenkopplung der Patienteninterfacevorrichtung 4 für die Glaskörper- und Retinachirurgie verwendet werden. In einer Ausführungsvariante ist die axiale Position (entlang der Projektionsachse z) des Linsensystems 44 manuell oder motorisch einstellbar. Im letzteren Fall umfasst die Patienteninterfacevorrichtung 4 einen Bewegungstreiber zum Verstellen des Linsensystems 44, welcher z.B. über eine Schnittstelle durch den Prozessor 1 2 ansteuerbar ist.

Wie in den Figuren 2 bis 6 schematisch dargestellt ist, umfasst die Patienteninterfacevorrichtung 4 eine transparente Schutzbarriere 46, die das Auge 2 vor einer direkten Kontaktierung durch das Linsensystem 44 oder damit verbundenen Verunreinigungen schützt. Im am Auge 2 befestigten Zustand der Patienteninterfacevorrichtung 4 liegt die Schutzbarriere 46 zwischen dem Linsensystem 44 und dem Auge 2. Die Schutzbarriere 46 ist eine flexible Folie oder Membran aus einem für das Auge 2 verträglichen Kunststoff, eine feste Glasscheibe oder ein gekrümmter Kontaktkörper, die überdies auch zum Applanieren der Cornea 20 eingerichtet sein kann.

In den folgenden Abschnitten wird näher auf den Prozessor 12 und die damit verbundenen optionalen funktionalen Module des ophthalmologischen Lasersystems 10 (optisches Übertragungssystem 16, Beam-Expander-Vorrichtung 17, Adaptionsoptik 18, optisches Messsystem 100, Konfokaldetektor 13, Kamera 14 und OCT-Vorrichtung 15) eingegangen, die in der Figur 6 schematisch in einem Blockdiagramm dargestellt sind, aber auch Teil der Laservorrichtung 1 sein können, die in den Figuren 2 bis 5 dargestellt ist.

Das optische Messsystem 100 ist eingerichtet, einen auf der zweiten Fokalfläche B* angeordneten Arbeitsbereich b* optisch entlang des Strahlengangs vom Projektionsobjektiv 30 zum Auge 2 zu ermitteln, abhängig von der Ausführungsvariante mittels des Konfokaldetektors 13, der Kamera 14 und/oder der OCT-Vorrichtung 15, wobei die Messsignale respektive Messdaten des optischen Messsystems 100 durch den Prozessor 12 entgegengenommen, gespeichert und verarbeitet werden.

Der optionale Konfokaldetektor 13 ist in den Strahlengang zwischen Laserquelle 11 und Scannvorrichtung 19 eingekoppelt und eingerichtet, Reflexionen des Laserstrahls L an Augengewebe zu erfassen, das in der Fokalfläche B* liegt. Mit anderen Worten, der Konfokaldetektor 13 ermöglicht die Detektion von reflektierenden Augenstrukturen, wenn diese in der Fokalfläche B* liegen. Zum Testen respektive Ausmessen einer konkreten Anordnung des ophthalmologischen Lasersystems 10, mit eingekoppeltem Linsensystem 44 und mittels der Befestigungsvorrichtung 43 am Auge 2 fixiert, wird ein Scannmuster mit reduzierter Leistung abgefahren, welche keine Gewebeverarbeitung bewirkt, und die Reflexionen am Gewebe werden über den Konfokaldetektor 13 erfasst. Dazu ist der Prozessor 12 eingerichtet, die Laserquelle 11 auf einen Testmodus mit der reduzierten Leistung zu setzen, und die Scannvorrichtung 18 so anzusteuern, dass diese den Fokus F bei der reduzierten Leistung gemäss einem Test-Scannmuster bewegt. Der Prozessor 12 speichert die vom Konfokaldetektor 13 während dem Abfahren des Test-Scannmusters erfassten und gelieferten Reflexionswerte zugeordnet zu den jeweils betreffenden Scannpunkten des Test-Scannmusters. Dadurch können auf der Fokalfläche B* liegende reflektierende Gewebestrukturen erfasst werden. In Kombination mit einer Fokusverstellung des Projektionsobjektivs 30 oder einer dem Konfokaldetektor 13 nachgeschalteten Zoomvorrichtung der Laservorrichtung 1 können die reflektierenden Gewebestrukturen mehrlagig auf Fokalflächen B* unterschiedlicher Tiefe erfasst und zu einer dreidimensionalen Abbildung respektive einem 3D-Modell der Augenstrukturen kombiniert werden. Der Konfokaldetektor 13 liefert den Vorteil, dass die Zielgenauigkeit des Lasersystems 10 nicht verändert wird durch ungenaue Montage der Vorrichtung (insbesondere von Applikationskopf 3 und Patienteninterfacevorrichtung 4) oder durch eine (manuelle oder motorische) Verschiebung des Linsensystems 44, die eine Verschiebung bzw. Verkippung der Bildebene b* bewirken können. Beispielsweise können sogenannte Floater sehr genau erfasst und sicher, selbst dicht an der Retina 22 (ohne deren Schädigung), durch Laserpulse aufgelöst werden.

Die Kamera 14, beispielsweise eine CCD-Kamera (Charged Coupled Device), ist eingerichtet, ein Aufsichtsbild des Arbeitsbereichs b* auf der Fokalfläche B* zu erfassen.

Die OCT-Vorrichtung 15 ist eingerichtet, den Arbeitsbereich b* auf der Fokalfläche B* und davor bzw. dahinter liegende Bereiche durch optische Kohärenztomografie zu erfassen. Dabei muss durch Verstellung eines Referenzarms der OCT-Vorrichtung 15 die zusätzliche optische Weglänge zwischen den Arbeitsbereichen b und b* berücksichtigt werden.

In einer Ausführungsvariante wird das optische Messsystem 100, insbesondere die Kamera 14 oder die OCT-Vorrichtung 15, zur Erfassung der Pupille 23 des Auges verwendet. Der Prozessor 12 ist eingerichtet, aus den vom optischen Messsystem 100 gelieferten Bilddaten respektive Messdaten der Pupille 23 die aktuelle Grösse der Pupille 23 zu ermitteln. Falls die ermittelte Pupillengrösse einen Durchmesserschwellwert unterschreitet, der eine Beschneidung der Strahlbreite des Laserstrahls L bewirken würde, steuert der Prozessor 12 die im Strahlengang dem Linsensystem 44 vorgelagerte Beam-Expander-Vorrichtung 17 an, die Strahlbreite des Laserstrahls L soweit zu reduzieren, bis er durch die Pupille 23, gemäss der ermittelten Pupillengrösse, keine Beschneidung der Strahlbreite mehr erfährt.

Die Adaptionsoptik 18 ist im Strahlengang dem Linsensystem 44 vorgelagert und umfasst einen oder mehrere deformierbare Spiegel, in den Strahlengang einbringbare Platten, LCD-Spiegel, und/oder Linsensysteme. Die Adaptionsoptik 18 ist eingerichtet, Aberrationen zu korrigieren, die bei einer Abbildung eines Fokus F des Laserstrahls L durch das Linsensystem 44 von der ersten Fokalfläche B auf die zweite Fokalfläche B* verursacht werden können. Die Adaptionsoptik 18 wird vom Prozessor 12 gesteuert, beispielsweise abhängig von Wellenfrontfehlern bei der Abbildung der ersten Fokalfläche B auf die zweite Fokalfläche B*, die in einer Kalibrierungsphase erfasst werden oder die dynamisch durch einen Wellenfrontsensor (nicht gezeichnet) ermittelt werden. Um Aberrationen aktiv auszugleichen, die durch die Vorrichtungsmontage bzw. das Auge 2 des Patienten verursacht werden, steuert der Prozessor 12 die Adaptionsoptik 18 unter Verwendung von Wellenfrontmessungen, die mit Hilfe des Wellenfrontsensors erfasst werden.

## Patentansprüche

1. Ophthalmologische Patienteninterfacevorrichtung (4), umfassend eine Kopplungsvorrichtung (41) zum mechanischen Ankoppeln der Patienteninterfacevorrichtung (4) an einen Applikationskopf (3) eines ophthalmologischen Lasersystems (10), wobei der Applikationskopf (3) ein Projektionsobjektiv (30) zum Fokussieren eines Laserstrahls auf eine erste Fokalfläche (B) umfasst, **dadurch gekennzeichnet,**
**dass** die Patienteninterfacevorrichtung (4) ein Linsensystem (44) umfasst, das, im am Applikationskopf (3) angekoppelten Zustand der Patienteninterfacevorrichtung (4) bei einer Behandlung eines Auges (2), zwischen dem Auge (2) und dem Applikationskopf (3) angeordnet ist, eingekoppelt in einen Strahlengang vom Projektionsobjektiv (30) des Applikationskopfes (3) zum Auge (2), wobei das Linsensystem (44) so eingerichtet ist, dass es die im Strahlengang dem Linsensystem (44) vorgelagerte erste Fokalfläche (B) des Projektionsobjektivs (30) an eine im Strahlengang dem Linsensystem (44) nachgeschaltete zweite Fokalfläche (B*) im Auge (2) abbildet, derart, dass der vom Projektionsobjektiv (30) auf die erste Fokalfläche (B) fokussierte Laserstrahl (L) in der zweiten Fokalfläche (B*) im Auge (2) eine Gewebebearbeitung bewirkt.

2. Ophthalmologische Patienteninterfacevorrichtung (4) nach Anspruch 1, **dadurch gekennzeichnet, dass** das Linsensystem (44) eine Relais-Optik umfasst, welche eingerichtet ist, die erste Fokalfläche (B) des Projektionsobjektivs (30) auf die zweite Fokalfläche (B*) im Auge (2) abzubilden.

3. Ophthalmologische Patienteninterfacevorrichtung (4) nach einem der Ansprüche 1 oder 2, **dadurch gekennzeichnet, dass** das Linsensystem (44) eingerichtet ist, einen ersten Bildbereich (b) auf der ersten Fokalfläche (B) des Projektionsobjektivs (30) auf einen bezüglich des ersten Bildbereichs (b) kleineren, zweiten Bildbereich (b*) auf der zweiten Fokalfläche (B*) im Auge (2) abzubilden.

4. Ophthalmologische Patienteninterfacevorrichtung (4) nach einem der Ansprüche 1 bis 3, **dadurch gekennzeichnet, dass** das Linsensystem (44) eingerichtet ist, die erste Fokalfläche (B) des Projektionsobjektivs (30) auf die zweite Fokalfläche (B*) im Auge (2) abzubilden, welche in einem Bereich von und mit Augenlinse (21) bis und mit Retina (22) liegt.

5. Ophthalmologische Patienteninterfacevorrichtung (4) nach einem der Ansprüche 1 bis 4, **dadurch gekennzeichnet, dass** die Patienteninterfacevorrichtung (4) so ausgestaltet ist, dass im am Applikationskopf (3) angekoppelten Zustand der Patienteninterfacevorrichtung (4) ein Zwischenraum (45) zwischen dem Projektionsobjektiv (30) des Applikationskopfes (3) und dem Linsensystem (44) besteht, welcher ein Gas, Gasgemisch oder Vakuum enthält, und dass die erste Fokalfläche (B) des Projektionsobjektivs (30) in den Zwischenraum (45) zu liegen kommt.

6. Ophthalmologische Patienteninterfacevorrichtung (4) nach Anspruch 5, **dadurch gekennzeichnet, dass** das Linsensystem (44) eingerichtet ist, die erste Fokalfläche (B) des Projektionsobjektivs (30) derart auf die zweite Fokalfläche (B*) im Auge (2) abzubilden, dass Aberrationen korrigiert werden, die aufgrund einer Fokussierung des Laserstrahls (L) auf die erste Fokalfläche (B) im Zwischenraum (45) mit Gas, Gasgemisch oder Vakuum anstatt einer Fokussierung des Laserstrahls (L) auf die erste Fokalfläche (B) in Augengewebe auftreten.

7. Ophthalmologische Patienteninterfacevorrichtung (4) nach einem der Ansprüche 5 oder 6, **gekennzeichnet durch** optisch erkennbare Referenzmarkierungen (48), welche im Zwischenraum (45) angeordnet sind.

8. Ophthalmologische Patienteninterfacevorrichtung (4) nach einem der Ansprüche 1 bis 7, **dadurch gekennzeichnet, dass** die Patienteninterfacevorrichtung (4) so ausgestaltet ist, dass, im am Applikationskopf (3) angekoppelten Zustand der Patienteninterfacevorrichtung (4), das Projektionsobjektiv (30) des Applikationskopfes (3) über dem Linsensystem (44) mechanisch frei verfahrbar ist.

9. Ophthalmologische Patienteninterfacevorrichtung (4) nach einem der Ansprüche 1 bis 8, **gekennzeichnet durch** eine Befestigungsvorrichtung (43) mit einer oder mehreren Unterdruckkammern (40) zum Befestigen der Patienteninterfacevorrichtung (4) am Auge (2), wobei das Linsensystem (44) auswechselbar mit der Befestigungsvorrichtung (43) verbunden ist.

10. Ophthalmologische Patienteninterfacevorrichtung (4) nach einem der Ansprüche 1 bis 9, **gekennzeichnet durch** eine transparente Schutzbarriere (26), die, im am Auge (2) befestigten Zustand der Patienteninterfacevorrichtung (4), zwischen dem Auge (2) und dem Linsensystem (44) angeordnet ist.

11. Ophthalmologisches Lasersystem (10), umfassend eine ophthalmologische Patienteninterfacevorrichtung (4) nach einem der Ansprüche 1 bis 10, eine Laserquelle (11) zur Erzeugung eines Laserstrahls (L), ein Projektionsobjektiv (30) zur fokussierten Projektion des Laserstrahls (L) auf einen Fokus (F) auf der ersten Fokalfläche (B), eine Scannvorrichtung (18) zum Bewegen des Fokus (F), **dadurch gekennzeichnet, dass** das ophthalmologische Lasersystem (10) zudem einen Konfokaldetektor (13) umfasst, welcher in den Strahlengang zwischen Laserquelle (11) und Scannvorrichtung (19) eingekoppelt ist, und welcher eingerichtet ist, eine Reflexion des Laserstrahls (L) durch in der zweiten Fokalfläche (B*) liegendes Augengewebe zu erfassen.

12. Ophthalmologisches Lasersystem (10) nach Anspruch 11, **gekennzeichnet durch** einen Prozessor (12), der eingerichtet ist, das ophthalmologische Lasersystem (10) derart zu steuern, dass die Laserquelle (11) mit reduzierter Leistung betrieben wird, die keine Gewebeverarbeitung bewirkt, und dass die Scannvorrichtung (18) den Fokus (F) bei der reduzierten Leistung gemäss einem definierten Scannmuster bewegt, und vom Konfokaldetektor (13) erfasste Werte der Reflexionen zugeordnet zu Punkten des Scannmusters zu speichern.

13. Ophthalmologisches Lasersystem (10) nach einem der Ansprüche 11 oder 12, **gekennzeichnet durch** ein optisches Messsystem (100), welches eingerichtet ist, einen auf der zweiten Fokalfläche (B*) angeordneten Arbeitsbereich (b*) optisch entlang des Strahlengangs vom Projektionsobjektiv (30) zum Auge (2) zu ermitteln, wobei das optische Messsystem (100) mindestens eines umfasst aus: eine Kamera (14), die eingerichtet ist, ein Aufsichtsbild des Arbeitsbereichs (b*) zu erfassen, und eine OCT-Vorrichtung (15), welche eingerichtet ist, den Arbeitsbereich (b*) durch optische Kohärenztomografie zu ermitteln.

14. Ophthalmologisches Lasersystem (10) nach einem der Ansprüche 11 bis 13, **gekennzeichnet durch** eine im Strahlengang dem Linsensystem (44) der ophthalmologischen Patienteninterfacevorrichtung (4) vorgelagerte Adaptionsoptik (18), welche eingerichtet ist, Aberrationen zu korrigieren, die bei einer Abbildung eines Fokus (F) des Laserstrahls (L) von der ersten Fokalfläche (B) auf die zweite Fokalfläche (B*) auftreten, wobei die Adaptionsoptik (18) mindestens eines umfasst aus: deformierbarer Spiegel, in den Strahlengang einbringbare Platte, LCD-Spiegel, und Linsensystem.

15. Ophthalmologisches Lasersystem (10) nach einem der Ansprüche 11 bis 14, **gekennzeichnet durch** ein optisches Messsystem (100), welches eingerichtet ist, eine Pupille (23) des Auges (2) optisch zu erfassen, einen Prozessor (12), der eingerichtet ist, eine Pupillengrösse zu ermitteln, und eine im Strahlengang dem Linsensystem (44) der ophthalmologischen Patienteninterfacevorrichtung (4) vorgelagerte Beam-Expander-Vorrichtung (17), welche eingerichtet ist, eine Strahlbreite des Laserstrahls (L) abhängig von der Pupillengrösse anzupassen.

## Claims

1. Ophthalmological patient interface apparatus (4), comprising a coupling apparatus (41) for mechanically coupling the patient interface apparatus (4) to an application head (3) of an ophthalmological laser system (10), wherein the application head (3) comprises a projection lens (30) for focussing a laser beam onto a first focal area (B), **characterized in that**
the patient interface apparatus (4) comprises a lens system (44) which, in a state where the patient interface apparatus (4) is coupled to the application head (3) during the treatment of an eye (2), is arranged between the eye (2) and the application head (3) and is coupled into a beam path from the projection lens (30) of the application head (3) to the eye (2), wherein the lens system (44) is configured to image the first focal area (B) of the projection lens (30), which is disposed upstream of the lens system (44) in the beam path, onto a second focal area (B*) in the eye (2) disposed downstream of the lens system (44) in the beam path, in such a way that the laser beam (L) focussed onto the first focal area (B) by the projection lens (30) causes tissue processing in the second focal area (B*) in the eye (2).

2. Ophthalmological patient interface apparatus (4) according to Claim 1, **characterized in that** the lens system (44) comprises relay optics configured to image the first focal area (B) of the projection lens (30) onto the second focal area (B*) in the eye (2) .

3. Ophthalmological patient interface apparatus (4) according to either of Claims 1 and 2, **characterized in that** the lens system (44) is configured to image a first image region (b) on the first focal area (B) of the projection lens (30) onto a second image region (b*), which is smaller than the first image region (b), on the second focal area (B*) in the eye (2) .

4. Ophthalmological patient interface apparatus (4) according to any one of Claims 1 to 3, **characterized in that** the lens system (44) is configured to image the first focal area (B) of the projection lens (30) onto the second focal area (B*) in the eye (2) disposed in a range from and including the eye lens (21) to and including the retina (22).

5. Ophthalmological patient interface apparatus (4) according to any one of Claims 1 to 4, **characterized in that**, in the state where the patient interface apparatus (4) is coupled to the application head (3), the patient interface apparatus (4) is configured such that an intermediate space (45) is present between the projection lens (30) of the application head (3) and the lens-element system (44), the intermediate space containing a gas, a gas mixture or a vacuum, and that the first focal area (B) of the projection lens (30) is brought into position in the intermediate space (45).

6. Ophthalmological patient interface apparatus (4) according to Claim 5, **characterized in that** the lens system (44) is configured to image the first focal area (B) of the projection lens (30) onto the second focal area (B*) in the eye (2) in such a way that aberrations are corrected, said aberrations occurring on account of focussing the laser beam (L) onto the first focal area (B) in the intermediate space (45) with a gas, a gas mixture or a vacuum instead of focussing the laser beam (L) onto the first focal area (B) in the eye tissue.

7. Ophthalmological patient interface apparatus (4) according to either of Claims 5 and 6, **characterized by** optically identifiable reference markings (48) which are arranged in the intermediate space (45).

8. Ophthalmological patient interface apparatus (4) according to any one of Claims 1 to 7, **characterized in that**, in the state where the patient interface apparatus (4) is coupled to the application head (3), the patient interface apparatus (4) is configured such that the projection lens (30) of the application head (3) is mechanically freely displaceable over the lens system (44).

9. Ophthalmological patient interface apparatus (4) according to any one of Claims 1 to 8, **characterized by** a fastening apparatus (43) with one or more negative pressure chambers (40) for fastening the patient interface apparatus (4) to the eye (2), wherein the lens system (44) is exchangeably connected to the fastening apparatus (43).

10. Ophthalmological patient interface apparatus (4) according to any one of Claims 1 to 9, **characterized in that**, in the state where the patient interface apparatus (4) is fastened to the eye (2), a transparent protection barrier (26) is arranged between the eye (2) and the lens system (44).

11. Ophthalmological laser system (10), comprising an ophthalmological patient interface apparatus (4) according to any one of Claims 1 to 10, a laser source (11) for producing a laser beam (L), a projection lens (30) for the focussed projection of the laser beam (L) onto a focus (F) on the first focal area (B), a scanning apparatus (18) for moving the focus (F), **characterized in that** the ophthalmological laser system (10) further comprises a confocal detector (13) which is coupled into the beam path between the laser source (11) and the scanning apparatus (19) and which is configured to capture a reflection of the laser beam (L) by eye tissue disposed in the second focal area (B*).

12. Ophthalmological laser system (10) according to Claim 11, **characterized by** a processor (12) which is configured to control the ophthalmological laser system (10) in such a way that the laser source (11) is operated with reduced power which does not cause tissue processing and that the scanning apparatus (18) moves the focus (F) in the case of the reduced power in accordance with a defined scanning pattern, and to store values of the reflections captured by the confocal detector (13) in a manner assigned to points of the scanning pattern.

13. Ophthalmological laser system (10) according to either of Claims 11 and 12, **characterized by** an optical measuring system (100) which is configured to optically determine a working region (b*) arranged on the second focal area (B*) along the beam path from the projection lens (30) to the eye (2), wherein the optical measuring system (100) includes at least one of: a camera (14) configured to capture a plan view image of the working region (b*) and an OCT apparatus (15) configured to determine the working region (b*) by optical coherence tomography.

14. Ophthalmological laser system (10) according to any one of Claims 11 to 13, **characterized by** adaptative optics (18) disposed upstream of the lens system (44) of the ophthalmological patient interface apparatus (4) in the beam path, the adaptive optics being configured to correct aberrations which occur during imaging of a focus (F) of the laser beam (L) from the first focal area (B) onto the second focal area (B*), wherein the adaptive optics (18) includes at least one of: a deformable mirror, a plate that is introducible into the beam path, an LCD mirror and a lens-element system.

15. Ophthalmological laser system (10) according to any one of Claims 11 to 14, **characterized by** an optical measuring system (100) which is configured to optically capture a pupil (23) of the eye (2), a processor (12) which is configured to determine a pupil size and a beam expander apparatus (17) disposed upstream of the lens system (44) of the ophthalmological patient interface apparatus (4) in the beam path, the beam expander apparatus being configured to adapt a beam width of the laser beam (L) depending on the pupil size.

## Revendications

1. Dispositif d'interface ophtalmologique (4) pour patients, comprenant un dispositif d'accouplement (41) pour l'accouplement mcanique du dispositif d'interface (4) pour patients une tte d'application (3) d'un systme laser ophtalmologique (10), la tte d'application (3) comprenant un objectif de projection (30) pour la focalisation d'un faisceau laser sur une premire surface focale (B), caractris en ce que le dispositif d'interface (4) pour patients comprend un systme de lentille (44) qui, dans l'tat du dispositif d'interface (4) pour patients accoupl la tte d'application (3) lors dun traitement d'un il (2), est dispos entre l'il (2) et la tte d'application (3), coupl dans une trajectoire de faisceau depuis l'objectif de projection (30) de la tte d'application (3) jusqu' l'il (2), le systme de lentille (44) tant conu de manire former limage de la premire surface focale (B) de l'objectif de projection (30) monte en amont du systme de lentille (44) dans la trajectoire du faisceau sur une deuxime surface focale (B*) dans l'il (2) en aval du systme de lentille (44) dans la trajectoire du faisceau, de telle sorte que le faisceau laser (L) focalis par l'objectif de projection (30) sur la premire surface focale (B) provoque un traitement des tissus dans l'il (2) dans la deuxime surface focale (B*).

2. Dispositif d'interface ophtalmologique (4) pour patients selon la revendication 1, caractris en ce que le systme de lentille (44) comprend une optique-relais qui est conue pour former limage de la premire surface focale (B) de l'objectif de projection (30) sur la deuxime surface focale (B*) dans l'il (2).

3. Dispositif d'interface ophtalmologique (4) pour patients selon l'une des revendications 1 ou 2, caractris en ce que le systme de lentille (44) est conu pour former limage dune premire rgion d'image (b) sur la premire surface focale (B) de l'objectif de projection (30) sur une deuxime rgion d'image (b*) plus petite par rapport la premire rgion d'image (b) sur la deuxime surface focale (B*) dans l'il (2).

4. Dispositif d'interface ophtalmologique (4) pour patients selon l'une des revendications 1 3, caractris en ce que le systme de lentille (44) est conu pour former limage de la premire surface focale (B) de l'objectif de projection (30) sur la deuxime surface focale (B*) dans l'il (2), laquelle deuxime surface focale est situe dans une rgion allant depuis le cristallin (21), cristallin inclus, jusqu' la rtine (22), rtine incluse.

5. Dispositif d'interface ophtalmologique (4) pour patients selon l'une des revendications 1 4, caractris en ce que le dispositif d'interface (4) pour patients est configur de telle sorte que dans l'tat du dispositif d'interface (4) pour patients accoupl la tte d'application (3), un espace intermdiaire (45) est prsent entre l'objectif de projection (30) de la tte d'application (3) et le systme de lentille (44), lequel contient un gaz, un mlange de gaz ou du vide, et en ce que la premire surface focale (B) de l'objectif de projection (30) est amene en position dans l'espace intermdiaire (45).

6. Dispositif d'interface ophtalmologique (4) pour patients selon la revendication 5, caractris en ce que le systme de lentille (44) est conu pour former limage de la premire surface focale (B) de l'objectif de projection (30) sur la deuxime surface focale (B*) dans l'il (2) de manire corriger les aberrations qui se produisent en raison d'une focalisation du faisceau laser (L) sur la premire surface focale (B) dans l'espace intermdiaire (45) comprenant du gaz, un mlange de gaz ou du vide, au lieu d'une focalisation du faisceau laser (L) sur la premire surface focale (B) dans les tissus de l'il.

7. Dispositif d'interface ophtalmologique (4) pour patients selon l'une des revendications 5 ou 6, caractris par des marquages de rfrence (48) pouvant tre dtects optiquement, lesquels sont disposs dans l'espace intermdiaire (45).

8. Dispositif d'interface ophtalmologique (4) pour patients selon l'une des revendications 1 7, caractris en ce que le dispositif d'interface (4) pour patients est configur de telle sorte que dans l'tat du dispositif d'interface (4) pour patients accoupl la tte d'application (3), l'objectif de projection (30) de la tte d'application (3) puisse tre dplac librement de manire mcanique par-dessus le systme de lentille (44).

9. Dispositif d'interface ophtalmologique (4) pour patients selon l'une des revendications 1 8, caractris par un dispositif de fixation (43) avec une ou plusieurs chambres de dpression (40) pour la fixation du dispositif d'interface (4) pour patients sur l'il (2), le systme de lentille (44) tant connect de manire changeable au dispositif de fixation (43).

10. Dispositif d'interface ophtalmologique (4) pour patients selon l'une des revendications 1 9, caractris par une barrire de protection transparente (26) qui, dans l'tat du dispositif d'interface (4) pour patients fix sur l'il (2), est dispose entre l'il (2) et le systme de lentille (44).

11. Systme laser ophtalmologique (10) comprenant un dispositif d'interface ophtalmologique (4) pour patients selon l'une des revendications 1 10, une source laser (11) pour gnrer un faisceau laser (L), un objectif de projection (30) pour la projection focalise du faisceau laser (L) sur un foyer (F) sur la premire surface focale (B), un dispositif de balayage (18) pour dplacer le foyer (F), caractris en ce que le systme laser ophtalmologique (10) comprend en outre un dtecteur confocal (13) qui est coupl dans la trajectoire du faisceau entre la source laser (11) et le dispositif de balayage (19), et qui est conu pour dtecter une rflexion du faisceau laser (L) par les tissus de l'il situs dans la deuxime surface focale (B*).

12. Systme laser ophtalmologique (10) selon la revendication 11, caractris par un processeur (12) qui est conu pour commander le systme laser ophtalmologique (10) de telle sorte que la source laser (11) fonctionne avec une puissance rduite qui ne provoque aucun traitement des tissus et que le dispositif de balayage (18) dplace le foyer (F) en cas de puissance rduite en fonction d'un modle de balayage dfini, et pour mmoriser des valeurs, dtectes par le dtecteur confocal (13), des rflexions associes des points du modle de balayage.

13. Systme laser ophtalmologique (10) selon l'une des revendications 11 ou 12, caractris par un systme de mesure optique (100) qui est conu pour dterminer optiquement une plage de travail (b*) dispose sur la deuxime surface focale (B*) le long de la trajectoire du faisceau de l'objectif de projection (30) vers l'il (2), le systme de mesure optique (100) comprenant au moins l'un parmi : une camra (14) qui est conue pour saisir une image visuelle de la plage de travail (b*), et un dispositif OCT (15) qui est conu pour dterminer la plage de travail (b*) par tomographie optique de cohrence.

14. Systme laser ophtalmologique (10) selon l'une des revendications 11 13, caractris par une optique d'adaptation (18) dispose dans la trajectoire du faisceau en amont du systme de lentille (44) du dispositif d'interface ophtalmologique (4) pour patients, laquelle est conue pour corriger des aberrations qui se produisent lors d'une reproduction d'un foyer (F) du faisceau laser (L) de la premire surface focale (B) sur la deuxime surface focale (B*), l'optique d'adaptation (18) comprenant au moins l'un parmi : un miroir dformable, une plaque pouvant tre introduite dans la trajectoire du faisceau, un miroir cristaux liquides, et un systme de lentille.

15. Systme laser ophtalmologique (10) selon l'une des revendications 11 14, caractris par un systme de mesure optique (100) qui est conu pour dtecter optiquement une pupille (23) de l'il (2), un processeur (12) qui est conu pour dterminer une taille de la pupille, et un dispositif de dilatation de faisceau (17) dispos dans la trajectoire du faisceau en amont du systme de lentille (44) du dispositif d'interface ophtalmologique (4) pour patients, qui est conu pour adapter une largeur de faisceau du faisceau laser (L) en fonction de la taille de la pupille.
